# EUROPEAN PATENT SPECIFICATION

(11) **EP 0 740 940 B1**
(45) Date of publication and mention of the grant of the patent: **16.06.2004**
(21) Application number: 96108547.9
(22) Date of filing: 06.08.1990
(51) Int. Cl.: A61L 2/18

(54) **Process for disinfecting bone tissue**
Verfahren zur Desinfektion von Knochengeweben
Procédé de désinfection des tissus os

(30) Priority: 09.08.1989 US 391233; 05.10.1989 US 417577
(43) Date of publication of application: 06.11.1996
(62) Divisional of application: 90912426.5
(73) Proprietor: OSTEOTECH, INC.,, Shrewsbury New Jersey 07702 (US)
(72) Inventor: O'Leary, Robert K., Spring Lake, New Jersey 07762 (US)
(74) Representative: LOUIS- PÖHLAU- LOHRENTZ

(56) References cited:
- WO-A-84/01894
- US-A- 4 315 919

## Description

This invention relates to the processing of bone tissue for use in grafts and transplants. More particularly, this invention relates to a process for preparing and disinfecting bone tissue under sterile conditions, employing a biocompatible surfactant, whereby there is improved penetration and diffusion of the biocompatible surfactant into the bone tissue.

In general, in processing musculoskeletal tissue for use in grafts and transplants, the musculoskeletal tissue, which may include, in addition to bone, connective tissue, such as fascia, tendons, ligaments, etc., is removed from the body under sterile conditions. The tissue is then soaked in an antibiotic and then wrapped under sterile conditions for transport to facilities for further processing.

Once the tissue is ready to be processed further, it is unwrapped under sterile conditions and, in the case of bone, then debrided; i.e., connective tissue and periosteum are removed from the bone. After debridement, the bone is shaped under sterile conditions into a specific size meeting formal specifications for surgical requirements.

After the bone has been shaped, it is treated with a disinfectant in order to kill vegetative organisms. After treatment with the disinfectant, the bone is then washed with sterile water and packaged under sterile conditions. Such tissue may be frozen until it is ready for use.

US 4,315,919 discloses a method for depyrogenating a proteinaceous biological or pharmaceutical product wherein pyrogens are lipopolysaccharides (LPS) derived from the outer cell wall of gram-negative bacteria.

WO 84/01894 is directed to a process for chemical sterilization of implantable biological tissue such as heart valves, ligaments, tendons, membranes contaminated with fungi or bacteria.

US 4,020,183 discloses a method of inactivating herpes simplex virus infectivity in human. Especially, US 4,020,183 is directed to the treatment of recurrent herpes labialis or recurrent herpetic vulvovaginitis.

It is an object of the present invention to provide a process whereby there is achieved improved penetration, diffusion, permeation, solubility, and sorption of a disinfectant into bone tissue during the preparation of such tissue for grafts and transplants.

It is also an object of the present invention to provide a process whereby viruses present in the bone tissue are killed during the preparation of such tissue for grafts and transplants.

In accordance with an aspect of the present invention, there is provided a process pursuant to claim 1.

Preferred embodiments are specified in dependent claims 2 to 5.

The antibiotics which may be employed are generally present in an aqueous solution. One or a combination of antibiotics may be used to treat the bone tissue in accordance with the present invention. Antibiotics which may be employed include bacitracin, polymyxin B sulfate, erythromycin, neomycin, penicillin, tetracyclines, viomycin, chloromycetin, streptomycins, cefazolin, ampicillin, tobramycin, clindamycin, and gentamicin. The antibiotic(s) is employed in an effective antibiotic amount. The term "antibiotic" as used herein means that the antibiotics produce effects adverse to the normal biological functions of organisms within the bone tissue, including death or destruction or prevention of the growth of such organisms.

The disinfectants which may be employed are generally present in an aqueous solution, and are administered in an effective disinfecting amount. Examples of disinfectants which may be employed include ethylene oxide, propylene oxide, ethanol, hydrogen peroxide (preferably as 10% hydrogen peroxide in aqueous solution), chlorine dioxide, chlorahexidene gluconate, glutaraldehyde, formaldehyde, peracetic acid (hydrogen peroxide and acetic acid in aqueous solution), povadone iodide (polyvinylpyrollidone), sodium hypochlorite, quaternary ammonium compounds , cetyl alcohol and benzalkonium chloride. A preferred disinfectant is an aqueous ethanol solution.

The disinfectants employed within the scope of the present invention will kill vegetative organisms, which may be remaining in the bone tissue. In addition, the disinfectants may also act as a viricide and kill viruses within the bone tissue as well. Ethanol, for example, has a viricidal effect upon a variety of viruses, including HIV, or AIDS, virus. Ethanol also functions in removing any fat which remains in the tissue.

It is to be understood that antibiotic(s) alone, disinfectant(s) alone, or a combination of antibiotic(s) and disinfectant(s) may be used in combination with the agent to increase diffusion of the treating agent(s) into the tissue.

The agent for increasing diffusion of the treating agent into the tissue is preferably a surfactant or a permeation enhancer. The surfactant employed in accordance with the present invention may be a cationic, non-ionic, anionic, or amphoteric surfactant. The surfactant should be a biocompatible surfactant and miscible with the disinfectant, such as ethanol, or with the antibiotic.

Cationic surfactants which may be employed include quaternary amino or nitrogen compounds; quaternary ammonium salts such as benzalkonium chloride, alkyltrimethylammonium salts, and alkylpyridinium salts; aliphatic mono-,di-, and polyamines; rosin-derived amines; amine oxides, such as polyoxyethylene alkyl and alicyclic amines, N, N, N, N tetrakis-substituted ethylene diamines, amide-linked amines, preferably those prepared by the condensation of a carboxylic acid with a di-or polyamine, and sodium tauro-24, 25-dihydrofusidate.

Anionic surfactants which may be employed include sulfates such as alkyl sulfates (for example, sodium dodecyl sulfate), sulfated fats and oils, sulfated oleic acid, sulfated alkanolamides, sulfated esters, and alcohol sulfates; sulfonates such as alkylaryl sulfonates, olefin sulfonates, ethoxylated alcohol sulfates, and sulfonates of ethoxylated alkyl phenols; sulfates of fatty esters; sulfates and sulfonates of alkyl phenols; lignosulfonates; sulfonates of condensed naphthalenes; sulfonates of naphthalene; dialkyl sulfosuccinates, preferably sodium derivatives; sodium derivatives of sulfo-succinates, such as the disodium ethoxylated nonyl phenol half ester of sulfosuccinic acid, the disodium ethoxylated alcohol (C₁₀- C₁₁), half-ester of sulfosuccinic acids, etc., petroleum.sulfonates, such as alkali salts of petroleum sulfonates; for example, sodium petroleum sulfonate (Acto 632); phosphate esters, such as alkali phosphate esters, and a potassium salt of phosphate ester (Triton H66); sulfonated alkyl esters (for example, Triton GR 7); carboxylates, such as those of the formula (RCOO)-(M)+ wherein R is an alkyl group having from 9-21 carbon atoms, and M is a metal or an amine; and sodium polymeric carboxylic acid (Tamol 731) and the like.

Nonionic surfactants which may be employed include polyoxyethylenes; ethoxylated alkyl phenols, ethoxylated aliphatic alcohols; carboxylic acid esters, such as glycerol esters, polyethylene glycol esters, and polyoxyethylene fatty acid esters; anhydrosorbitol esters and ethoxylated anhydrosorbitol esters; glycol esters of fatty acids; ethoxylated natural fats, oils, and waxes; carboxylic amides, such as diethanolamine condensates, and monoalkanolamine condensates; polyoxyethylene fatty acid amides; polyalkylene oxide block copolymers, preferably polyethylene and polypropylene oxide block copolymers; and polysiloxane-polyoxyalkylene copolymers; 1-dodecylazacycloheptan-2-one (Nelson R & D); polyethylene glycol monolaurate (Alza); and Macrochem's SEPA nonionic surfactant.

Preferred non-ionic surfactants are non-ionic surfactants which are ethylene oxide condensation products (polyoxyethylene) containing more than two, and preferably at least five ethylene oxide groups, with at least one end group thereof being terminated by condensation with either an alcohol, alkylphenol, or a long chain fatty acid. A particularly preferred non-ionic surfactant is an octylphenoxy polyethoxyethanol surfactant known as Triton X-100.

Amphoteric surfactants include N-coco-3 aminopropionic acid and its sodium salt; disodium salts of N-tallow-3-iminodipropionate and N-lauryl-3-iminodipropionate; N-carboxymethyl-N cocoalkyl-N-dimethylammonium hydroxide; N-carboxymethyl-N-dimethyl-N-(9-octadecenyl) ammonium hydroxide; (1-carboxy heptadecyl) trimethylammonium hydroxide; (1-carboxyundecyl) trimethylammonium hydroxide; sodium salts of N-cocoamidoethyl-N-hydroxyethylglycine and N-hydroxyethyl-N-stearamido-glycine; sodium salts of N-hydroxyethyl-N-lauramido-B-alanine and N-cocoamido-N-hydroxyethyl-B-alanine; sodium salts of mixed alicyilic amines, ethoxylated and sulfated sodium salts or free acids of 2-alkyl-1 carboxymethyl-1-hydroxyethyl-2-imidazolinium hydroxide; the disodium salt of 1, 1-bis (carboxymethyl)-2-undecyl-2-imidazolinium hydroxide; and the sodium salt of a propoxylated and sulfated oleic acid-ethylenediamine condensate.

In lieu of a surfactant, it is to be understood that a permeation enhancer may be employed as the agent to increase diffusion of the at least one treating agent (disinfectant and/or antibiotic) into the bone tissue. The term "permeation enhancer" as used in the art and herein means an agent which aids or assists a material (e.g., a drug) to dissolve into and diffuse through the skin. Applicant has found that such permeation enhancers may be employed to increase diffusion of a treating agent into bone tissue.

Permeation enhancers which may be employed within the scope of the present invention include glycerol monolaurate; hexamethylene lauramide; dimethyl formamide; propylene glycol; diethyltoluamide; N-methyl-2-pyrrolidone; decylmethylsulfoxide; benzyl alcohol; dimethyl sulfoxide; alkyl-N-N-dialkyl-substituted amino acetates; lecithin; dimethylacetamide; laurocapram; dodecyl-L-pyroglutamate; 1-oxohydrocarbyl-substituted azacyclohexanes; azone; hydroxyethyl acetamide; tetrahydrofurfuryl alcohol; methyl laurate; isopropyl palmitate; isopropyl myristate; and isopropyl stearate. Preferred permeation enhancers are isopropyl palmitate and isopropyl myristate. It is to be understood, however, that the scope of the present invention is not to be limited to the specific permeation enhancers hereinabove described.

The disinfectant is employed alone or in an aqueous solution. Preferably, the disinfectant is added in an aqueous solution in an amount of from about 1% to about 100%. The agent to increase diffusion of the treating agent into the tissue, such as a surfactant or permeation enhancer, is added in an amount effective to increase diffusion of the disinfectant into the tissue. The agent is preferably added to an aqueous solution of the disinfectant in an amount of from about 0.0001% to about 10% of the resulting aqueous solution, preferably from about 0.01% to about 10%. Wherein an antibiotic is included as a treating agent, the antibiotic(s) is preferably added in an aqueous solution, in an amount amount of from about 30,000 units to about 50,000 units, and the agent to increase diffusion of the treating agent (i.e., the antibiotic) into the tissue, such as a surfactant or permeation enhancer, is added to the aqueous solution in an amount of from about 0.0001% to about 10% of the resulting aqueous solution, (preferably from about 0.01% to about 10%. The combination of disinfectant or antibiotic(s) and surfactant or permeation enhancer is preferably applied to the tissue as an aqueous mixture.

Although the present invention is not intended to be limited to any theoretical reasoning, it is believed that, when a surfactant is employed as the agent to increase diffusion of the treating agent into the tissue, the combination of disinfectant and/or antibiotic and surfactant alters the surface tension of the cells of the bone tissue, thereby providing for improved or enhanced penetration of the disinfectant or antibiotic into and diffusion of the disinfectant or antibiotic through the tissue. The improved absorption of the disinfectant or antibiotic into the tissue provides for increased killing of bacteria, vegetative organisms, and/or viruses which may be remaining in the bone tissue. In addition, the use of a surfactant can provide viricidal activity in that surfactants can function to disrupt viruses.

When a permeation enhancer is employed as the agent to increase diffusion of the treating agent into the tissue, it is believed that the permeation enhancer increases the solubility of the antibiotic and/or disinfectant; i.e., the permeation enhancer increases the ability of the antibiotic and/or disinfectant to dissolve into the musculoskeletal tissue. It is also believed that the permeation enhancer increases the diffusion, or rate of movement, of the antibiotic and/or disinfectant through the tissue.

Tissue which is disinfected in accordance with the present invention is bone tissue. Specific examples of bone tissue which may be prepared for use in grafts or transplants in accordance with the present invention include proximal tibia, distal femur, proximal humerus, (with or without rotator cuff), proximal femur, whole.knee, whole femur, fibula, ribs, femoral heads, fibular shafts, femoral shafts, and tibial shafts. The tissue may be from a human or non-human animal.

In accordance with the present invention, there is provided a process for treating bone tissue which comprises contacting bone tissue with an effective viricidal amount of a surfactant. The term "viricidal" as used herein means that the surfactants employed in the present invention inhibit, prevent, or destroy the growth or proliferation of viruses.

The surfactant employed in accordance with the present invention may be a cationic, non-ionic, anionic, or amphoteric surfactant, as hereinabove described. The surfactant is a biocompatible surfactant. A preferred surfactant is a non-ionic surfactant, and most preferably an octylphenoxy polyethoxy ethanol surfactant.

Applicant has surprisingly found that a surfactant, such as those hereinabove described, is able, by altering the surface tension of the cells of the bone tissue, to penetrate into and diffuse through the bone tissue such that viruses are effectively killed upon contact of the surfactant with the bone tissue. The surfactant, upon penetration into and diffusion through the bone tissue, is able to function to disrupt viruses.

The surfactant contacts the bone tissue in an effective viricidal amount. In a preferred embodiment, the surfactant is added in an aqueous solution in an amount of from ab out 0.0001% to about 10%, most preferably from about 0.01% to about 10%.

Tissue which may be treated with the surfactant in accordance with this aspect of the present invention including the bone tissue hereinabove described.

It is to be understood that the processes of the present invention are carried out in vitro; i.e., the processes are undertaken after the bone tissue is removed from the body.

It is to be understood, however, that the scope of the present invention is not to be limited to the specific embodiments described above. The invention may be practiced other than as particularly described and still be within the scope of the accompanying claims.

## Claims

1. A process for disrupting, in vitro, virus in bone tissue, of bone selected from the group
consisting of proximal tibia, distal femur, proximal humerus, proximal femur, whole knee, whole femur, fibula, ribs, fibular shafts, femoral shafts, and tibial shafts, said process comprising:
Disrupting virus in said bone tissue by contacting said bone with an effective viricidal amount of a biocompatible surfactant, whereby said biocompatible surfactant penetrates into said one tissue to disrupt viruses in said bone tissue.

2. The process of Claim 1 wherein the biocompatible surfactant is a non-ionic surfactant.

3. The process of Claim 2 wherein the non-ionic surfactant is an octylphenoxy polethoxy ethanol surfactant.

4. The process of any of Claims 1 to 3 wherein the biocompatible surfactant is added in an aqueous solution in an amount of from about 0.0001 % to about 10 %.

5. The process of Claim 4 wherein the biocompatible surfactant is added in an amount of from about 0.01 % to about 10 %.

## Patentansprüche

1. Verfahren zum in vitro-Aufbrechen eines Virus im Knochengewebe von Knochen, die aus der Gruppe proximales Schienbein, distaler Oberschenkelknochen, proximaler Oberarmknochen, proximaler Oberschenkelknochen, gesamtes Knie, gesamter Oberschenkelknochen, Wadenbein, Rippen, Fibulaschäfte, Femurschäfte und Tibiaschäfte ausgewählt sind, wobei das Verfahren folgendes umfasst:
Aufbrechen des Virus im genannten Knochengewebe durch Kontaktieren des Knochens mit einer wirksamen virizidalen Menge eines bioverträglichen oberflächenaktiven Mittels, wobei dieses bioverträgliche oberflächenaktive Mittel in dieses eine Gewebe eindringt, um die Viren in diesem Knochengewebe aufzubrechen.

2. Verfahren nach Anspruch 1, wobei es sich bei dem bioverträglichen oberflächenaktiven Mittel um ein nicht-ionisches oberflächenaktives Mittel handelt.

3. Verfahren nach Anspruch 2, wobei es sich bei dem nicht-ionischen oberflächenaktiven Mittel um ein Octylphenoxypolyethoxyethanol-Tensid handelt.

4. Verfahren nach einem der Ansprüche 1 bis 3, wobei das bioverträgliche oberflächenaktive Mittel in einer wässrigen Lösung in einer Menge von etwa 0,0001 bis etwa 10 % zugesetzt wird.

5. Verfahren nach Anspruch 4, wobei das bioverträgliche oberflächenaktive Mittel in einer Menge von etwa 0,01 bis etwa 10 % zugesetzt wird.

## Revendications

1. Un procédé pour combattre, in vitro, un virus dans un tissu osseux d'un os sélectionné dans le groupe constitué par le tibial proximal, le fémur distal, l'humérus proximal, le fémur proximal, le genou entier, le fémur entier, le péroné, les côtes, la hampe du péroné, les hampes fémorales et les hampes du tibia, ce procédé consistant à perturber le virus présent dans ledit tissu osseux en amenant ledit os au contact d'une quantité effectivement viricide d'un surfactif biocompatible, ledit surfactif biocompatible pénétrant dans ledit tissu de manière à combattre lesdits virus dans ledit tissu osseux.

2. Le procédé de la revendication 1, dans lequel le surfactif biocompatible est un surfactif non-ionique.

3. Le procédé de la revendication 2, dans lequel le surfactif non-ionique est un surfactif du type octyl-phénoxy-polyéthoxy-éthanol.

4. Le procédé selon l'une quelconque des revendications 1 à 3, dans lequel le surfactif biocompatible est ajouté en solution aqueuse à raison d'une quantité allant d'environ 0,0001 % jusqu'à environ 10 %.

5. Le procédé de la revendication 4, dans lequel le surfactif biocompatible est ajouté à raison d'une quantité allant d'environ 0,01 % jusqu'à 10 %.
